# EUROPEAN PATENT SPECIFICATION

(11) **EP 2 687 162 B1**
(45) Date of publication and mention of the grant of the patent: **03.06.2015**
(21) Application number: 12757435.8
(22) Date of filing: 15.03.2012
(51) Int. Cl.: A61B 8/00, A61B 5/00

(54) **PHOTOACOUSTIC IMAGE GENERATING DEVICE AND METHOD**
PHOTOAKUSTISCHE BILDERZEUGUNGSVORRICHTUNG UND VERFAHREN DAFÜR
PROCÉDÉ ET DISPOSITIF DE GÉNÉRATION D'IMAGES PHOTO-ACOUSTIQUES

(30) Priority: 16.03.2011 JP 2011057747; 06.03.2012 JP 2012048784
(43) Date of publication of application: 22.01.2014
(73) Proprietor: FUJIFILM Corporation, Tokyo 106-8620 (JP)
(72) Inventor: TSUJITA, Kazuhiro, Ashigarakami-gun Kanagawa 258-8538 (JP); WADA, Takatsugu, Ashigarakami-gun Kanagawa 258-8538 (JP)
(74) Representative: Klunker . Schmitt-Nilson . Hirsch
(86) International application number: PCT/JP2012/001821
(87) International publication number: WO 2012/124341

(56) References cited:
- EP-A1- 1 493 380
- WO-A1-02/15776
- WO-A1-2009/154298
- WO-A2-2007/100937
- JP-A- 10 052 428
- JP-A- 2004 147 940
- JP-A- 2005 021 380
- JP-A- 2006 246 974
- JP-A- 2009 142 320
- US-A1- 2009 187 099

## Description

### Technical Field

The present invention is related to a photoacoustic image generating apparatus and a photoacoustic image generating method. More specifically, the present invention is related to a photoacoustic image generating apparatus and a photoacoustic image generating method that irradiate a laser beam onto a subject, detect ultrasonic waves generated within the subject due to the irradiation of the laser beam, and generate photoacoustic images.

### Background Art

The ultrasound examination method is known as an image examination method that enables examination of the state of the interior of living organisms in a non invasive manner. Ultrasound examination employs an ultrasound probe capable of transmitting and receiving ultrasonic waves. When the ultrasonic waves are transmitted to a subject (living organism) from the ultrasound probe, the ultrasonic waves propagate through the interior of the living organisms, and are reflected at interfaces among tissue systems. The ultrasound probe receives the reflected ultrasonic waves and images the state of the interior of the subject, by calculating distances based on the amounts of time that the reflected ultrasonic waves return to the ultrasound probe.

Photoacoustic imaging, which images the interiors of living organisms utilizing the photoacoustic effect, is also known. Generally, in photoacoustic imaging, pulsed laser beams are irradiated into living organisms. Biological tissue within the living organisms that absorbs the energy of the pulsed laser beams generates ultrasonic waves (photoacoustic signals) by adiabatic expansion thereof. An ultrasound probe or the like detects the photoacoustic signals, and constructs photoacoustic images based on the detected signals, to enable visualization of the living organisms based on the photoacoustic signals.

In photoacoustic imaging, it is necessary for comparatively high output laser beams to be irradiated into living organisms. From the viewpoint of safety, it is preferable for the output of the pulsed laser beams to be prevented when a probe is not in contact with the living organisms. In this regard, Patent Document 1 discloses an apparatus provided with a measurement target detecting means that detects measurement targets along an optical path, that irradiates light when the measurement target detecting means detects a measurement target. The properties of the measurement targets can be utilized to detect the measurement targets. Specifically, the light shielding properties, the reflectivities, unique temperatures, weights, and electrostatic capacities of the measurement targets can be utilized to detect the measurement targets.

### [Background Art Documents]

### [Patent Documents]

[Patent Document 1]
   Japanese Unexamined Patent Publication No. 2009-142320

A photoacoustic image generating apparatus according to the preamble of claim 1 is also known from document WO 2007/100937.

### Disclosure of the Invention

When generating photoacoustic images, it is necessary to irradiate light onto a location at which it is desired for a photoacoustic image to be generated and then to detect photoacoustic waves generated at the location. The measurement target detecting means of Patent Document 1 merely detects whether a measurement target is positioned at a position onto which a laser beam is to be output, and cannot detect onto what portion of the measurement target the laser beam will be irradiated. In the invention of Patent Document 1, laser emission is enabled if the measurement target is set. Therefore, there are cases in which the laser beam is irradiated onto a position other than a desired position, and photoacoustic images are generated at positions other than those that are intended to be imaged.

The present invention has been developed in view of the foregoing circumstances. It is an object of the present invention to provide a photoacoustic image generating apparatus and a photoacoustic image generating method that can prevent light such as laser beams from being irradiated onto positions other than that at which a photoacoustic image is to be generated.

In order to achieve the above object, the present invention provides a photoacoustic image generating apparatus, comprising:
a light source unit that outputs light to be irradiated onto a subject;
a probe that transmits acoustic waves toward the subject, detects photoacoustic signals which are generated within the subject due to irradiation of the light, and detects reflected acoustic signals of the transmitted acoustic waves;
probe position judging means for comparing the features of reflected acoustic signals detected by the probe against the features of reflected acoustic signals, which are detected in advance by the probe while the probe is at a position suited for generating a photoacoustic image, and for judging whether the compared features match;
a light source control means for instructing the light source unit to output light when the probe position judging means judges that the compared features match; and
photoacoustic image generating means for generating photoacoustic images based on photoacoustic signals detected by the probe.

A configuration may be adopted, wherein the photoacoustic image generating apparatus of the present invention further comprises:
acoustic wave image generating means for generating acoustic wave images based on the reflected acoustic signals; and wherein:
   the probe position judging means utilizes the acoustic wave images to compare the features of the reflected acoustic signals. In this case, the probe position judging means may extract features from the generated acoustic wave images, and compare the extracted features against the features of the reflected acoustic signals which are obtained in advance.

A configuration may be adopted, wherein the photoacoustic image generating apparatus of the present invention further comprises:
reference image storing means for storing an acoustic wave image generated by the acoustic wave image generating means based on reflected acoustic signals detected by the probe while it is at a position suited for generation of a photoacoustic image as a reference image; and wherein:
   the probe position judging means extracts features from the stored reference image, and compares the extracted features of the acoustic wave images.

The photoacoustic image generating apparatus of the present invention may further comprise:
contact state judging means for judging whether the probe is in contact with the subject; and
light irradiation suppressing means for suppressing output of the light when the contact state judging means judges that the probe is not in contact with the subject. In this case, the contact state judging means may judge whether the probe is in contact with the subject based on reflected acoustic signals detected by the probe.

It is desirable for the probe of the photoacoustic image generating apparatus of the present invention to comprise:
a backing material;
a plurality of inorganic piezoelectric elements arranged on the surface of the backing material;
a first acoustic matching layer provided on the plurality of inorganic piezoelectric elements; and
a second acoustic matching layer provided on the first acoustic matching layer;
the second acoustic matching layer comprising an upper side organic layer that constitutes a plurality of organic piezoelectric elements and a lower side organic layer that performs acoustic matching with respect to the plurality of inorganic piezoelectric elements with the upper side organic layer.

Alternatively, a configuration may be adopted, wherein the probe comprises:
a plurality of inorganic piezoelectric elements arranged on a backing material; and
a plurality of organic piezoelectric elements provided along with the inorganic piezoelectric elements in a direction parallel to the surface of the backing material.

In each of the probes described above, it is desirable for the plurality of inorganic piezoelectric elements to be employed to transmit acoustic waves; and for the plurality of organic piezoelectric elements to be employed to detect reflected acoustic waves.

The present invention also provides a photoacoustic image generating method, comprising the steps of:
transmitting acoustic waves from a probe toward a subject;
detecting reflected acoustic signals of the transmitted acoustic waves with the probe;
comparing the features of reflected acoustic signals detected by the probe against the features of reflected acoustic signals, which are detected in advance by the probe while the probe is at a position suited for generation of a photoacoustic image, and judging whether the compared features match;
irradiating light onto the subject when it is judged that the compared features match in the comparing step;
detecting photoacoustic signals generated within the subject due to irradiation of the light with the probe; and
generating photoacoustic images based on the detected photoacoustic signals.

Note that it is desirable for the photoacoustic image generating method of the present invention to further comprise the step of:
generating acoustic wave images based on the reflected acoustic signals; and wherein:
   the acoustic wave images are utilized to compare the features of the reflected acoustic signals in the comparing step.

In this case, it is desirable for features to be extracted from the generated acoustic wave images, and for the extracted features to be compared against the features of the reflected acoustic signals which are obtained in advance in the comparing step.

In the case that the extracted features are employed in the comparison as described above, it is desirable to store an acoustic wave image generated by the acoustic wave image generating means based on reflected acoustic signals detected by the probe while it is at a position suited for generation of a photoacoustic image as a reference image; for features to be extracted from the stored reference image, and for the extracted features to be employed for comparison in the comparing step.

It is desirable for the photoacoustic image generating method of the present invention to further comprise the steps of:
judging whether the probe is in contact with the subject; and
suppressing output of the light when it is judged that the probe is not in contact with the subject.

In such a case, it is preferable for judgment regarding whether the probe is in contact with the subject to be performed based on reflected acoustic signals detected by the probe.

The photoacoustic image generating apparatus and the photoacoustic image generating method of the present invention transmits and receives acoustic waves at a position where a photoacoustic image is to be generated in advance, and detects reflected acoustic waves at this position. When generating a photoacoustic image, acoustic waves are transmitted and received with respect to a subject prior to irradiating light, and whether the features of detected reflected acoustic waves and the features of the reflected acoustic waves which were detected in advance match is judged. Light is irradiated onto the subject, photoacoustic signals are detected, and a photoacoustic image is generated if it is judged that the features match. By adopting this configuration, photoacoustic images can be generated at desired positions at which acoustic waves were detected in advance, and wasteful irradiation of light onto positions other than that at which a photoacoustic image is to be generated can be prevented.

### Brief Description of the Drawings

Figure 1 is a block diagram that illustrates a photoacoustic image generating apparatus according to a first embodiment of the present invention.
Figure 2 is a flow chart that illustrates operational procedures when registering a position.
Figure 3 is a flow chart that illustrates operational procedures when generating a photoacoustic image.
Figure 4 is a block diagram that illustrates a photoacoustic image generating apparatus according to a second embodiment of the present invention.
Figure 5 is a partially exploded perspective view that illustrates an example of a probe.
Figure 6 is a side sectional view that illustrates a portion of the probe of Figure 5.
Figure 7A is a schematic diagram that illustrates a method for producing the ultrasound probe of Figure 5.
Figure 7B is a schematic diagram that illustrates the method for producing the ultrasound probe of Figure 5.
Figure 7C is a schematic diagram that illustrates the method for producing the ultrasound probe of Figure 5.
Figure 7D is a schematic diagram that illustrates the method for producing the ultrasound probe of Figure 5.
Figure 7E is a schematic diagram that illustrates the method for producing the ultrasound probe of Figure 5.
Figure 8 is a diagram that illustrates a drive circuit of the probe of Figure 5.
Figure 9 is a partially exploded perspective view that illustrates another example of a probe.

### Best Mode for Carrying out the Invention

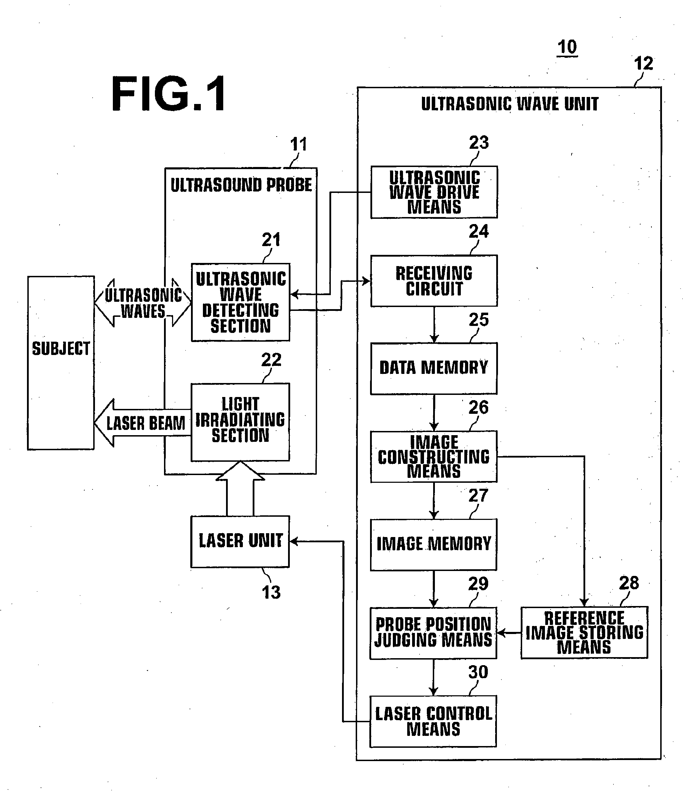

Hereinafter, embodiments of the present invention will be described in detail with reference to the attached drawings. Figure 1 illustrates a photoacoustic image generating apparatus 10 according to a first embodiment of the present invention. The photoacoustic image generating apparatus 10 includes: an ultrasound probe (probe) 11; an ultrasonic wave unit 12; and a light source (laser unit) 13. The laser unit 13 outputs a laser beam to be irradiated onto subject. The wavelength of the light beam to be irradiated onto subjects may be set as appropriate according to targets of observation. The laser beam output by the laser unit 13 is guided to the probe 11 by a light guiding means such as an optical fiber.

The probe 11 includes an ultrasonic wave detecting section 21 that detects ultrasonic waves as an example of acoustic waves and a light irradiating section 22. The light irradiating section 22 irradiates the laser beam output by the laser unit 13 toward subjects. The ultrasonic wave detecting section 21 outputs (transmits) ultrasonic waves toward subjects and detects (receives) acoustic waves reflected by the subjects. The probe 11 has a plurality of ultrasonic transducers which are arranged one dimensionally, for example. The ultrasonic wave detecting section 21 detects two types of ultrasonic waves (ultrasonic signals). The first type is ultrasonic waves (hereinafter, also referred to as "photoacoustic signals") which are generated by targets of measurement within subjects absorbing the laser beam output by the laser unit 13. The second type is reflected ultrasonic waves (hereinafter, also referred to as "reflected acoustic signals") of ultrasonic waves output toward subjects. Note that the light irradiating section 22 may be provided separately from the probe 11.

The ultrasonic wave unit 12 has an ultrasonic wave drive means 23, a receiving circuit 24, a data memory 25, an image constructing means 26, an image memory 27, a reference image storing means 28, a probe position judging means 29, and a laser control means 30. The ultrasonic wave drive means 23 drives the plurality of ultrasonic transducers included in the ultrasonic wave detecting section 21 of the probe 11, and causes the ultrasonic wave detecting section 21 to output ultrasonic waves. The ultrasonic wave drive means 23 causes the ultrasonic transducers to output ultrasonic waves by outputting pulsed electrical signals having a predetermined waveform to the plurality of ultrasonic transducers. The receiving circuit 24 receives ultrasonic signals (photoacoustic signals or reflected acoustic signals) detected by the ultrasonic wave detecting section 21. The receiving circuit 24 includes an A/D converter that samples the ultrasonic wave signals and converts the ultrasonic wave signals to digital signals, for example. The receiving circuit stores (sampled data of) the received ultrasonic wave signals in the data memory 26.

The image constructing means 26 generates tomographic images based on ultrasonic wave signals. The image constructing means 26 reads out photoacoustic signals from the data memory 25 and generates photoacoustic images based on the read out photoacoustic signals, for example. In addition, the image constructing means 26 reads out reflected acoustic signals from the data memory 25 and generates ultrasound images based on the read out reflected acoustic signals. The image constructing means 26 stores the generated photoacoustic images and the generated ultrasound images in the image memory 27. Note that in Figure 1, the image constructing means 26 generates both photoacoustic images and ultrasound images. Alternatively, a photoacoustic image generating means that generates photoacoustic images and an ultrasound image generating means that generates ultrasound images may be provided as separate means.

The probe position judging means 29 compares the features of reflected acoustic signals detected by the probe against the features of reflected acoustic signals obtained in advance by the probe 11 detecting reflected acoustic signals while it is at a position suited for generation of a photoacoustic image. In the present embodiment, the probe position judging means 29 does not directly employ the reflected acoustic signals, but utilizes ultrasound images which are generated based on reflected acoustic signals to compare the features of the ultrasound images (reflected acoustic signals). Examples of the features of the ultrasound image include: the presence or absence of blood vessels; the presence, absence, or position of a boundary of the epidermis; and the depth and the position at which sufficiently strong signals can be obtained. The probe position judging means 29 extracts features from the ultrasound images stored in the image memory 27, and compares the extracted features against the features of an ultrasound image which is generated based on the reflected acoustic signals detected by the probe 11 while it is at a position suited for generation of a photoacoustic image.

The reference image storing means 28 has stored therein an ultrasound image which is generated by the image constructing means 26 based on reflected acoustic signals detected by the probe 11 when the probe 11 is at a position suited for generation of a photoacoustic image as a reference image. The probe position judging means 29 extracts features from the stored reference image, and compares the features extracted from the reference image against features which are extracted from a current ultrasound image generated by the image constructing means 26, for example. As an alternative to this configuration, features extracted from the reference image may be stored, and the store features may be compared against features which are extracted from a current ultrasound image generated by the image constructing means 26. The laser control means 30, which is a light source control means, outputs a signal that commands light output to the laser unit 13 when the probe position judging means 29 judges that the features match.

The operational procedures of the photoacoustic image generating apparatus 10 will be described. A user registers a position suited for generating a photoacoustic image in advance. The user instructs the photoacoustic image generating apparatus 10 to register a position suited for generating a photoacoustic image. The user moves the probe 11 to a position suited for generating a photoacoustic image (step A1). After the probe 11 is moved, the ultrasonic wave drive means 23 supplies a drive signal to the ultrasonic transducers of the ultrasonic wave detecting section 21 of the probe 11, and causes ultrasonic waves to be transmitted from the probe 11 to a subject (step A2). The ultrasonic wave detecting section 21 detects reflected acoustic signals of the transmitted ultrasonic waves (step A3). The receiving circuit 24 stores the detected reflected acoustic signals in the data memory 25.

The image constructing means 26 reads out the reflected acoustic signals from the data memory 25, and generates an ultrasound image (step A4). The reference image storing means 28 stores the ultrasound image generated at step A4 as a reference image (step A5). In the case that there are a plurality of positions at which photoacoustic images are to be generated, the procedures of step A1 through step A5 are repeatedly executed, and ultrasound images of a plurality of positions may be stored as reference images. Preliminary preparations for generating a photoacoustic image are completed by storing the reference images.

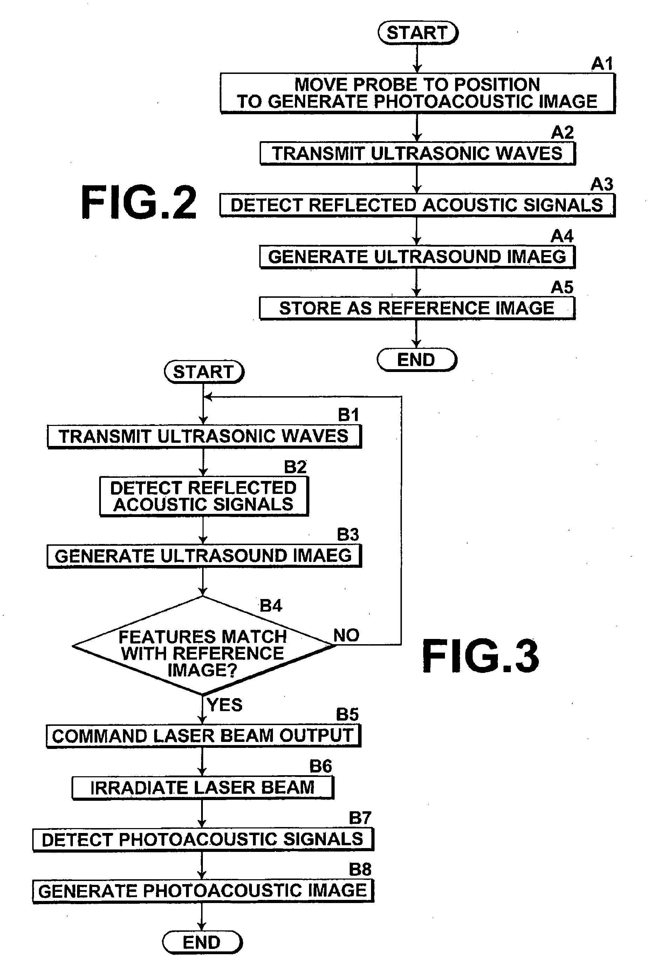

Figure 3 illustrates the operational procedures when a photoacoustic image is generated. After registering a position, the user commands photoacoustic image generation in a state in which the probe 11 is at an arbitrary position. The user switches the mode of the photoacoustic image generating apparatus 10 to that which is capable of generating photoacoustic images by operating a manual switch, footswitch, or the like. Alternatively, the switch may be performed automatically. The ultrasonic wave drive means 23 drives the probe 11 and causes ultrasonic waves to be transmitted toward a subject (step B1). The ultrasonic wave detecting section 21 of the probe 11 detects reflected acoustic signals of the ultrasonic waves that were transmitted at step B1 (step B2). The receiving circuit 24 stores the detected reflected acoustic signals in the data memory 25.

The image constructing means 26 reads out the reflected acoustic signals from the data memory 25, and generates an ultrasound image (step B3). The image constructing means 26 stores the generated ultrasound image in the image memory 27. The probe position judging means 29 compares the ultrasound image stored in the reference image storing means 28 as a reference image and the current ultrasound image stored in the image memory 27, and judges whether the features of the two images match (step B4). If the features do not match, the probe position judging means 29 judges that the probe 11 is not at the position which was registered as the position suited for generating a photoacoustic image, or at a position having the same features as the position suited for generating a photoacoustic image. In this case, the process returns to step B1. the photoacoustic image generating apparatus 10 repeatedly executes the procedures from step B1 through step B4 until it is judged that the features match at step B4.

If it is judged that the features match at step B4, it is judged that the probe 11 is at the position which was registered as the position suited for generating a photoacoustic image, or at a position having the same features as the position suited for generating a photoacoustic image. In this case, the laser control means 30 outputs a signal that commands laser beam output to the laser unit 13 (step B5). The laser unit 13 outputs a laser beam in response to the signal from the laser control means 30. The laser beam output from the laser unit 13 is irradiated onto the subject from the light irradiating section 22 of the probe 11 (step B6). The ultrasonic wave detecting section 21 detects photoacoustic signals which are generated within the subject due to irradiation of the laser beam (step B7). The receiving circuit 24 stores the detected photoacoustic signals in the data memory 25. The image constructing means 26 reads out the photoacoustic signals from the data memory 25, and generates a photoacoustic image (step B8). The generated photoacoustic image is displayed by an image display means (not shown), for example.

In the present embodiment, ultrasonic waves are transmitted and received at a position where a photoacoustic image is to be generated in advance, and an ultrasound image at this position is stored as a reference image. When generating a photoacoustic image, ultrasonic waves are transmitted and received with respect to a subject prior to irradiating a laser beam, and a generated ultrasound image and the ultrasound image stored as a reference image are compared. The ultrasonic wave unit 12 commands the laser unit 13 to output a laser beam when the features of the two ultrasound images match. The present embodiment judges whether the probe 11 is at a position which is registered in advance or at a position having features similar to those of the registered position. The laser beam is irradiated onto a subject after it is confirmed that the probe 11 is at the position which is registered in advance or at a position having features similar to the registered position. Thereby, photoacoustic images can be generated by irradiating the laser beam at a desired position, and wasteful irradiation of light and generation of photoacoustic images at positions other than that at which a photoacoustic image is to be generated can be prevented.

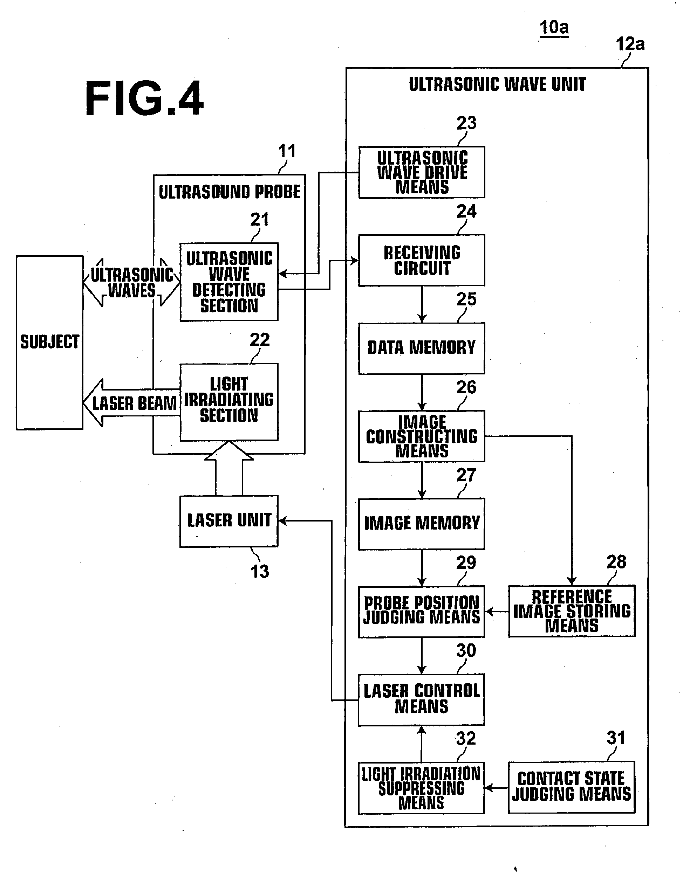

Next, a second embodiment of the present invention will be described. Figure 4 illustrates a photoacoustic image generating apparatus 10a according to the second embodiment of the present invention. The photoacoustic image generating apparatus 10a of the present embodiment differs from the photoacoustic image generating apparatus 10 of the first embodiment illustrated in Figure 1 in that an ultrasonic wave unit 12a further includes a contact state judging means 31 and a light irradiation suppressing means 32. The other constituent elements of the photoacoustic image generating apparatus 10a may be the same as those of the first embodiment. Note Figure 4 illustrates a case in which the contact state judging means 31 and the light irradiation suppressing means 32 are provided within the ultrasonic wave unit 12a. Alternatively, one or both of these elements may be provided within the probe 11.

The contact state judging means 31 judges whether the probe 11 is in contact with a subject. Means such as a pressure sensor that detects that the probe 11 is in contact with a subject may be employed as the contact state judging means 31. In this case, the contact state judging means 31 may judge the contact state of the probe 11 and the subject based on the amount of detected pressure. As an alternative to employing a physical sensor, the contact state judging means 31 may judge the contact state based on ultrasound images generated by the image constructing means 26. For example, the contact state judging means 31 may judge that the probe 11 is not in contact with a subject when an ultrasound image is that of an image pattern that typically appears when the probe 11 is not in contact with the subject, specifically, when high brightness lines parallel to the ultrasonic transducers are included in the ultrasound image in the vicinity of the ultrasonic transducers.

When the probe 11 is separated from a subject, the contact state judging means 31 outputs a signal indicating the separation to the light irradiation suppressing means 32. The light irradiation suppressing means 32 suppresses irradiation of a laser beam onto the subject when the contact state judging means 31 judges that the probe 11 is not in contact with the subject. For example, the light irradiation suppressing means 32 outputs a laser suppression signal to the laser control means 30. The laser control means 30 will not output a signal to command output of a laser beam to the laser unit 13 if the laser suppression signal is received. Thereby, irradiation of a laser beam onto the subject can be prevented. As an alternative to suppressing output of the laser irradiation command to the laser unit, irradiation of a laser beam onto the subject may be prevented by providing a means for blocking an output laser beam in an optical path between the laser unit 13 and the subject.

In the present embodiment, the light irradiation suppressing means 32 prevents irradiation of a laser beam onto subjects when the probe 11 is separated from the subjects. By adopting this configuration, situations in which laser beams are emitted into space and enter human eyes can be avoided, and safety with respect to eyes can be improved. The other advantageous effects are the same as those obtained by the first embodiment.

Note that the procedures from transmission of ultrasonic waves to comparison of the features of ultrasound images (steps B1 through B4) illustrated in Figure 3 need not be performed each time that a photoacoustic image is generated. In the case that photoacoustic images are repeatedly generated, for example, a photoacoustic image may be generated after executing the procedures of transmitting and receiving ultrasonic waves to comparing the features of ultrasound images only for a first photoacoustic image generating operation, and steps B1 through B4 may be omitted during subsequent photoacoustic image generating operations. Alternatively, steps B1 through B4 may be executed at a rate of once every plurality of photoacoustic image generating operations, to intermittently confirm the position of the probe 11.

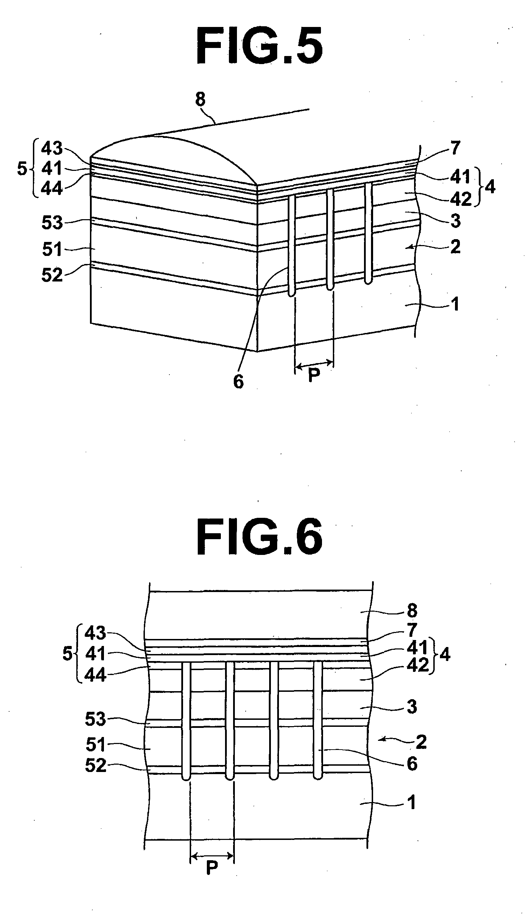

Here, examples of probes (ultrasound probes) that may be employed in the photoacoustic image generating apparatus of the present invention instead of the probe 11 will be described in detail. First, the probe illustrated in Figure 5 and Figure 6 will be described. A plurality of inorganic piezoelectric elements 2 are arranged and formed on the surface of a backing material 1 at a predetermined pitch P. The inorganic piezoelectric elements 2 that will function as ultrasonic transducers have a plurality of inorganic piezoelectric bodies 51 which are separated from each other. A signal electrode layer 52 is joined to one surface of each inorganic piezoelectric body 51, and a grounding electrode layer 53 is joined to the other surface of each inorganic piezoelectric body 51. That is, each inorganic piezoelectric element 2 is formed by a dedicated inorganic piezoelectric body 51, a signal electrode layer 52, and a grounding electrode layer 53.

A first acoustic matching layer 3 is joined on the plurality of inorganic piezoelectric elements 2. The first acoustic matching layer 3 is divided into a plurality of pieces and are arranged at the same pitch P as that of the plurality of inorganic piezoelectric elements 2.

A second acoustic matching layer 4 is joined on the first acoustic matching layer 3. The second acoustic matching layer 4 has an upper side organic layer 41 and a lower side organic layer 42.

The lower side organic layer 42 is divided into a plurality of pieces and are arranged on the first acoustic matching layer 3 at the same pitch P as that of the plurality of inorganic piezoelectric elements 2. Meanwhile, the upper side organic layer 41 is not divided, and extends over the entirety of the lower side organic layer 42. The sum of the thickness of the upper side organic layer 41 and the thickness of the lower side organic layer 42 is a thickness that satisfies λ/4 resonance conditions with respect to a wavelength A of a fundamental wave of ultrasonic waves transmitted by the plurality of inorganic piezoelectric elements 2. The upper side organic layer 41 and the lower side organic layer 42 together acoustically match the ultrasonic waves transmitted by the plurality of inorganic piezoelectric elements 2.

Further, the upper side organic layer 41 constitutes a portion of a plurality of organic piezoelectric elements 5. That is, a grounding electrode layer 43 is joined to the upper side organic layer 41 such that it extends over the surface thereof. In addition, a plurality of signal electrode layers 44, which are divided at the same pitch P as that of the plurality of inorganic piezoelectric elements 2, are joined to the surface of the upper side organic layer 41 that faces the lower side organic layer 42. Thereby, the upper side organic layer 4 functions as the plurality of organic piezoelectric elements 5. Each of the organic piezoelectric elements 5 which are arranged and formed in this manner is constituted by a dedicated signal electrode layer, an upper side organic layer which is common to the plurality of organic piezoelectric elements 5, and a grounding electrode layer 43. For this reason, the arrangement pitch of the organic piezoelectric elements 5 is determined only by the arrangement pitch of the plurality of signal electrode layers 44 which are joined to the underside of the upper side electrode layer 43, and the plurality of organic piezoelectric elements 5 are arranged at the same pitch P as that of the plurality of inorganic piezoelectric elements 2.

In addition, the plurality of pieces of the inorganic piezoelectric elements 2, the first acoustic matching layer 3, the lower side organic layer 42 of the second acoustic matching layer 4 and the signal electrode layer 44 which are divided at the same pitch P are positioned and aligned in the stacking direction thereof among each layer, and a filling agent 6 fills the gaps thereamong. That is, the filling agent 6 repeatedly fills the gaps at the same pitch P such that it penetrates through each layer from the surface of the signal electrode layer 44 to the surface of the backing material 1. Further, an acoustic lens 8 is coupled onto the plurality of organic piezoelectric elements 5 via a protective layer 7.

The inorganic piezoelectric bodies 51 of the inorganic piezoelectric elements 2 are formed by a piezoelectric ceramic exemplified by lead zirconate titanate (PZT™) or a piezoelectric single crystal exemplified by a lead magnesium niobate-titanate solid solution (PMN-PT). Meanwhile, the upper side organic layer 41 of the organic piezoelectric elements 5 is formed by a polymer piezoelectric element such as polyvinylidene fluoride (PVDF) and polyvinylidene fluoride trifluoroethylene copolymer (P(VDF-TrFE)).

The backing material supports the plurality of inorganic piezoelectric elements 2 and absorbs ultrasonic waves which are discharged toward the rear, and may be formed by a rubber material such as ferrite rubber.

The first acoustic matching layer 3 is provided to enable ultrasonic waves generated by the plurality of inorganic piezoelectric elements 2 to efficiently enter subjects. The first acoustic matching layer 3 is formed by a material having an acoustic impedance value intermediate the acoustic impedance value of the inorganic piezoelectric elements 2 and the acoustic impedance value of living tissue.

The second first acoustic matching layer 4 is provided to enable ultrasonic beams generated by the plurality of inorganic piezoelectric elements 2 to efficiently enter subjects. The lower side organic layer 42 maybe formed by a polymer piezoelectric element such as polyvinylidene fluoride (PVDF) and polyvinylidene fluoride trifluoroethylene copolymer (P(VDF-TrFE)) similarly to the upper side organic layer 4. Note that it is preferable for the upper side organic layer 41 and the lower side organic layer 42 to be formed by materials having the same or approximate acoustic impedances. If the acoustic impedance of the upper side organic layer and the acoustic impedance of the lower side organic layer are within a range of ±10% with respect to each other, the second acoustic matching layer 4 can be constituted without influencing acoustic matching of ultrasonic waves.

The filling agent 6 is provided to fix the positions and orientations of adjacent pieces, and is formed by epoxy resin or the like.

The protective layer 7 protects the grounding electrode layer 43 of the organic piezoelectric elements 5, and is formed by polyvinylidene fluoride (PVDF), for example.

The acoustic lens 8 focuses ultrasonic wave beams utilizing refraction to improve resolution in an elevation direction, and is formed by silicone rubber or the like.

Next, the operation of this probe will be described. During operation, the plurality of inorganic piezoelectric elements are utilized as dedicated ultrasonic wave transmitting transducers, and the plurality of organic piezoelectric elements 5 are utilized as dedicated ultrasonic wave receiving transducers, for example.

A pulsed voltage or continuous wave voltage is applied between the signal electrode layers 52 and the grounding electrode layers 53 of the plurality of inorganic piezoelectric elements 2. The inorganic piezoelectric bodies 51 of the inorganic piezoelectric elements 2 expand and contract due to the applied voltage, and pulsed ultrasonic waves or continuous wave ultrasonic waves are generated. The ultrasonic waves enter a subject via the first acoustic matching layer 3, the second acoustic matching layer 4, the protective layer 7, and the acoustic lens 8, are combined to form a ultrasonic wave beam, and propagate within the subject.

Reflected ultrasonic waves which are reflected within the subject enter the organic piezoelectric elements 5 via the acoustic lens 8 and the protective layer 7. When the reflected ultrasonic waves enter the piezoelectric elements 5, the upper organic layer 41 expands and contracts in response to harmonic components of the ultrasonic waves at high sensitivity, and electrical signals are generated between the signal electrode layer and the grounding electrode layer 43. The electrical signals are output as received signals.

An ultrasound image may be generated based on the received signals output by the plurality of organic piezoelectric elements 5. The generated ultrasound image may be employed in the feature comparison described above. Here, the plurality of inorganic piezoelectric elements and the plurality of organic piezoelectric elements 5 are arranged and formed at the same pitch P. Therefore, reflected ultrasonic waves from the subject can be received at the same arrangement positions as the transmission positions of transmitted ultrasonic waves, and high precision ultrasound images can be generated.

Note that the plurality of inorganic piezoelectric elements 2 may be utilized both as ultrasonic wave transmitting transducers and as ultrasonic wave receiving transducers. In this case, the reflected ultrasonic waves received by the organic piezoelectric elements 5 via the acoustic lens 8 and the protective layer 7 further enter the inorganic piezoelectric elements 2 via the second acoustic matching layer 4 and the first acoustic matching layer 1. The inorganic piezoelectric elements 2 expand and contract mainly in response to the fundamental wave components of the ultrasonic waves, and electrical signals are generated between the signal electrode layers 52 and the grounding electrode layers 53.

A compound ultrasound image, in which fundamental wave components and harmonic wave components are combined, may be generated based on received signals corresponding to the fundamental wave components obtained from the plurality of inorganic piezoelectric elements 2 and received signals corresponding to the harmonic wave components obtained from the plurality of organic piezoelectric elements 5.

In this case as well, the plurality of inorganic piezoelectric elements and the plurality of organic piezoelectric elements 5 are arranged and formed at the same pitch P. Therefore, fundamental wave components and harmonic wave components of reflected ultrasonic waves from the subject can be received at the same arrangement positions, and compound ultrasound images in which the fundamental wave components and the harmonic wave components are combined with high precision can be generated.

The probe described above may be produced in the following manner.

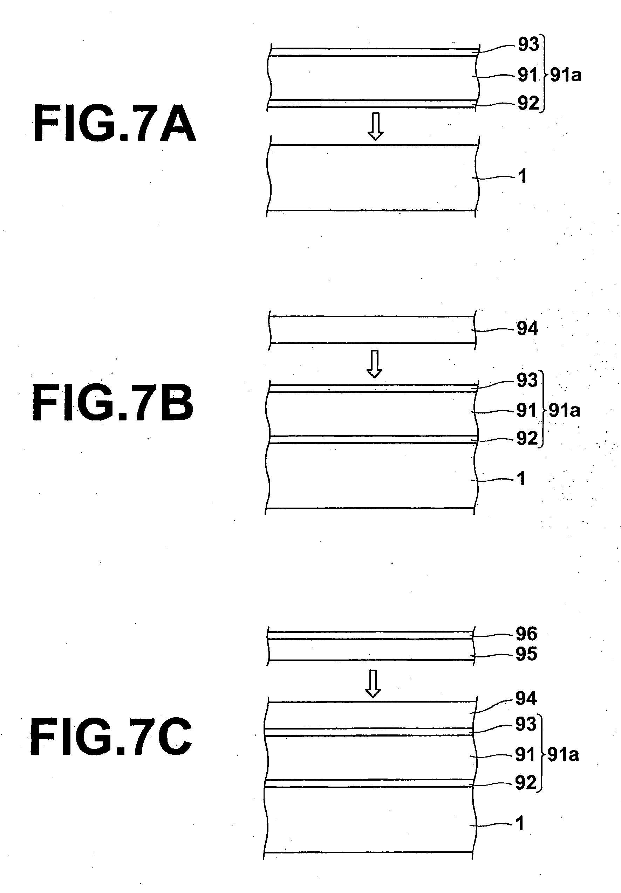

First, as illustrated in Figure 7A, an inorganic piezoelectric element layer 91a that extends across the entire surface of the backing material 1 is attached to the surface of the backing material 1 with an adhesive or the like. The inorganic piezoelectric element layer 91a has a conductive layer 92 and a conductive layer 93 formed on the entireties of the two surfaces thereof.

Next, as illustrated in Figure 7B, an acoustic matching layer 94 that extends across the entire region of the inorganic piezoelectric element layer 91a is joined to the conductive layer 93 at a temperature within a range from 120°C to 150°C. Then, as illustrated in Figure 7C, an organic layer 95 is joined on the acoustic matching layer 94. The organic layer 95 has a size that extends across the entire surface of the acoustic matching layer 94, and has a conductive layer 96 across the entire surface thereof opposite the surface that faces the acoustic matching layer 94.

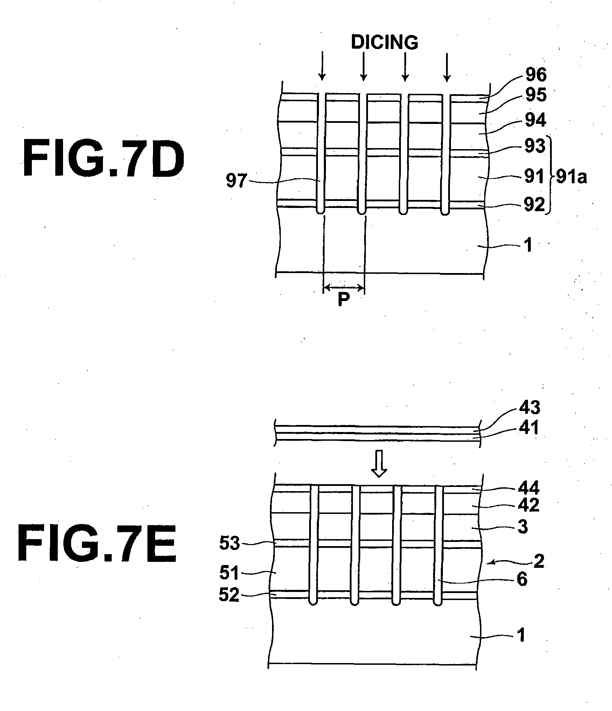

Next, as illustrated in Figure 7D, the conductive layer 96, the organic layer 95, the acoustic matching layer 94, and the inorganic piezoelectric element layer 91a are diced at a pitch P, to divide each layer into a plurality of pieces. At this time, dicing is performed to completely separate each layer from the conductive layer 96 through the inorganic piezoelectric element layer 91a. Therefore, the divided pieces of each layer are positionally aligned. Thereby, a plurality of inorganic piezoelectric elements 2 are formed on the surface of the backing material 1 at the arrangement pitch P. Pieces of the first acoustic matching layer 3, the lower side organic layer 42, and the signal electrode layer 44 are formed on the inorganic piezoelectric elements 2 such that they sequentially overlap thereon. In addition, a plurality of grooves 97 that penetrate through each layer in the stacking direction are formed between each column of pieces of each layer, which are aligned at the pitch P.

By dicing each layer from the conductive layer 9 through the inorganic piezoelectric element layer 91a at the pitch P, each layer is divided into a plurality of pieces in a simple manner, and each of the pieces of the divided layers can be positionally aligned in the stacking direction. In addition, the signal electrode layers 44 of the plurality of organic piezoelectric elements 5 and the signal electrode layers 52 and the grounding electrode layers 53 of the plurality of inorganic piezoelectric elements 2 can be accurately positionally aligned.

Next, the filling agent 6 fills the plurality of grooves 97 which are formed by dicing, and the positions and orientations of the pieces of each layer are fixed, as illustrated in Figure 7E. Thereafter, the upper side organic layer 41 is pressed onto the plurality of signal electrode layers 44 at a temperature of approximately 80°C. The upper side organic layer 41 has a size that extends across the entirety of the plurality of signal electrode layers 44, and the grounding electrode layer 43 is formed in advance across the entirety of the surface of the upper side organic layer 41 opposite the surface thereof that faces the plurality of signal electrode layers 44.

Here, the upper side organic layer 41 constitutes a portion of the second acoustic matching layer 4 for acoustically matching ultrasonic waves which are transmitted from the plurality of inorganic piezoelectric elements 2. The sum of the thickness of the upper side organic layer 41 and the thickness of the lower side organic layer 42 is a thickness that satisfies λ/4 resonance conditions with respect to a wavelength λ of a fundamental wave of ultrasonic waves transmitted by the plurality of inorganic piezoelectric elements 2. If only the upper side organic layer 41 is considered, the thickness thereof is not limited by the resonance conditions. Therefore, the electrical capacitance of the organic piezoelectric elements 5 can be increased by forming the upper side organic layer 41 to be thinner. That is, the upper side organic layer 41 may be formed to be of a desired thickness that yields an electrical capacitance that enables reflected ultrasonic waves received by the organic piezoelectric elements 5 to be efficiently converted into received signals. The lower side organic layer 42 may be formed such that the sum of the thickness of the upper side organic layer 41 and the thickness of the lower side organic layer 42 satisfies the aforementioned resonance conditions. Thereby, the plurality of organic piezoelectric elements 5 can be formed to be thin, while the second acoustic matching layer 4 satisfies resonance conditions with respect to the inorganic piezoelectric elements 2.

Here, the degree of crystallization of the upper side organic layer 41 gradually decreases accompanying increases in temperature. Therefore, the utilization upper limit temperature is considerably lower than the Curie point. For example, if the upper organic layer 41 is exposed to high temperature within the range from 80°C to 100°C which is utilized when laminating the layers such as the acoustic matching layer 94, depolarization will occur. However, the upper organic layer 41 is laminated after the other layers excluding the protective layer 7 and the acoustic lens 8 are laminated. For this reason, the upper organic layer 41 is not exposed to the high temperatures which are utilized when laminating the other layers and when the filling agent 6 fills the grooves 97. Thereby, depolarization can be suppressed.

Further, the upper organic layer 41 is not present when the layers under the upper organic layer 41, that is, the signal electrode layer 52, the inorganic piezoelectric body 51, the grounding electrode layer 53, the first acoustic matching layer 3, the lower side organic layer 42, and the signal electrode layer 44, are sequentially joined. Therefore, these layers can be joined at high temperatures and laminated with high adhesive force.

After the upper organic layer 41 is laminated on the plurality of signal electrode layers 44 in this manner, the acoustic lens 8 is joined with the grounding electrode layers 43 of the plurality of organic piezoelectric elements 5 via the protective layer 7, and the probe illustrated in Figure 5 and Figure 6 is completed.

In the case that a linear probe, in which the frequency of ultrasonic waves transmitted by the plurality of inorganic piezoelectric elements 2 is approximately 7MHz, the acoustic impedance of the first acoustic matching layer 3 is approximately 8.9rayl (kg/m²s), and the acoustic impedance of the second acoustic matching layer 4 is approximately 4.0rayl (kg/m²s), PZT™ may be utilized as the material of the inorganic piezoelectric bodies 51. The inorganic piezoelectric bodies 51 formed by PZT™ may be formed to have a thickness of approximately 190µm, and the first acoustic matching layer 3 may be formed to have a thickness of approximately 80µm. PVDF may be utilized as the material of the lower side organic layer 42 and the upper side organic layer 41. The lower side organic layer 42 may be formed to have a thickness of approximately 60µm, and the upper side organic layer 42 may be formed to have a thickness of approximately 20µm, such that the thickness of the second acoustic matching layer 4 as a whole becomes approximately 80µm. Thereby, the plurality of organic piezoelectric elements 5 can be formed at a desired thickness while the second acoustic matching layer 4 satisfies resonance conditions with respect to the plurality of inorganic piezoelectric elements 2.

The second acoustic matching layer 4 is of a two layer structure that includes the upper side organic layer 41 and the lower side organic layer 42. The second acoustic matching layer 4 is formed such that the upper side organic layer 41 that constitutes the plurality of organic piezoelectric elements 5 are formed to have a desired thickness while the sum of the thickness of upper organic layer 41 and the thickness of the lower organic layer 42 satisfies resonance conditions with respect to the plurality of inorganic piezoelectric elements 2. Thereby, the conversion efficiency of the plurality of organic piezoelectric elements 5 with respect to received signals can be improved while maintaining a superior acoustic transmission rate with respect to ultrasonic waves transmitted by the plurality of inorganic piezoelectric elements 2.

In addition, the plurality of inorganic piezoelectric elements 2 and the plurality of organic piezoelectric elements 5 are arranged in positional alignment, high precision compound ultrasound images can be generated.

Further, the upper side organic layer 41 that functions as the organic piezoelectric bodies of the organic piezoelectric elements 5 is not exposed to high temperatures during manufacture of the ultrasound probe. Therefore, depolarization of the upper side organic layer 41 can be suppressed.

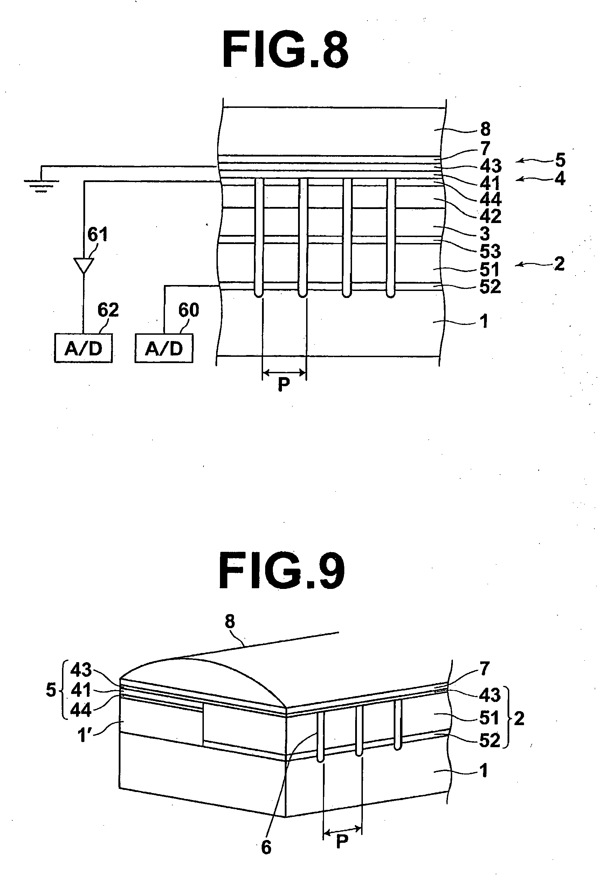

Note that an A/D converter 60 for inorganic piezoelectric elements may be connected to the signal electrode layer 52 of each inorganic piezoelectric element 2, and an amplifier 61 for organic piezoelectric elements and an A/D converter 62 for organic piezoelectric elements may be connected to the signal electrode layer 44 of each organic piezoelectric element 5, as illustrated in Figure 8.

Here, the electrical capacities of the plurality of organic piezoelectric elements 5 can be increased by setting the thicknesses of the organic piezoelectric bodies thereof to be thin as described above. However, it is difficult to obtain received signals having sufficient intensity only by such a configuration, and it is necessary to amplify received signals with the amplifier 61 for organic piezoelectric elements. At this time, it is preferable for the amplifier 61 for organic piezoelectric elements to be connected close to the signal electrode layers 44 of the organic piezoelectric elements 5 or directly connected thereto, in order to prevent attenuation of received signals while the received signals are transmitted from the organic piezoelectric elements 5 to the amplifier 61 for organic piezoelectric elements.

A multiplexer may be provided in the probe to reduce the number of signal lines which are drawn out from the probe. For example, a multiplexer may be provided in a signal chain after the A/D converter 60 for inorganic piezoelectric elements and the A/D converter 62 for organic piezoelectric elements. Thereby, two signal lines originating from the A/D converter 60 for inorganic piezoelectric elements and the A/D converter 62 for organic piezoelectric elements can be reduced to a single signal line.

In addition, the lower organic layer 95 having the conductive layer 96 formed on the surface thereof in advance was laminated on the first acoustic matching layer 94. However, the present invention is not limited to this configuration. The lower side organic layer 95 may be laminated onto the first acoustic matching layer 94, and then the conductive layer 96 may be formed on the surface of the lower side organic layer 95 thereafter.

Similarly, the upper side organic layer 41 having the grounding electrode layer 43 formed on the surface thereof in advance was laminated on the plurality of signal electrode layers 42. Alternatively, the upper side organic layer 41 may be jointed to the plurality of signal electrode layers 42, and then the grounding electrode layer 43 may be formed on the surface of the upper side organic layer 41 thereafter.

Next, another example of a probe (ultrasound probe) that may be employed by the photoacoustic image generating apparatus of the present invention will be described with reference to Figure 9. Note that in Figure 9, elements which are the same as those illustrated in Figure 5 and Figure 6 are denoted by the same reference numerals, and detailed descriptions thereof will be omitted insofar as they are not particularly necessary.

In the probe of Figure 9, the plurality of inorganic piezoelectric elements 2 for transmitting ultrasonic waves and the plurality of organic piezoelectric elements for receiving reflected ultrasonic waves are provided not in a stacked manner but arranged in directions parallel to the surface of the backing material. Note that in the present example, the grounding electrode layer 43 of the organic piezoelectric elements 5 also serve as the grounding electrode layer of the inorganic piezoelectric elements 2. In addition, in the present example, a second backing material 1' is laminated as a tier on the backing material 1, and the organic piezoelectric elements 5 are formed on the second backing material 1'. Alternatively, the backing material 1 may be processed to form a tiered shape.

In the configuration described above, ultrasonic waves emitted by the inorganic piezoelectric elements 2 are irradiated onto subjects without passing through the organic piezoelectric elements 5. In the case that a probe having this configuration as well, ultrasound images may be generated based on received signals output from the plurality of organic piezoelectric elements, and the generated ultrasound images may be employed in the feature comparison described above.

Preferred embodiments of the present invention have been described above. However, the photoacoustic image generating apparatus and the photoacoustic image generating method of the present invention are not limited to the above embodiments. Various changes and modifications to the configurations of the above embodiments are included in the scope of the present invention.

## Claims

1. A photoacoustic image generating apparatus (10), comprising:
a light source unit (13) that outputs light to be irradiated onto a subject;
a probe (11) that transmits acoustic waves toward the subject, detects photoacoustic signals which are generated within the subject due to irradiation of the light, and detects reflected acoustic signals of the transmitted acoustic waves; photoacoustic image generating means (26) for generating photoacoustic images based on photoacoustic signals detected by the probe (11); **characterized by**
probe position judging means (29) for comparing the features of reflected acoustic signals detected by the probe (11) against the features of reflected acoustic signals, which are detected in advance by the probe (11) while the probe (11) is at a position at which a photoacoustic image is to be generated, and for judging whether the compared features match; and
a light source control means (30) for instructing the light source unit (13) to output light when the probe position judging means (29) judges that the compared features match.

2. A photoacoustic image generating apparatus (10) as defined in Claim 1, further comprising:
acoustic wave image generating means (26) for generating acoustic wave images based on the reflected acoustic signals; and wherein:
the probe position judging means (29) utilizes the acoustic wave images to compare the features of the reflected acoustic signals.

3. A photoacoustic image generating apparatus (10) as defined in Claim 2, wherein:
the probe position judging means (29) extracts features from the generated acoustic wave images, and compares the extracted features against the features of the reflected acoustic signals which are obtained in advance.

4. A photoacoustic image generating apparatus (10) as defined in Claim 3, further comprising:
reference image storing means (28) for storing an acoustic wave image generated by the acoustic wave image generating means (26) based on reflected acoustic signals detected by the probe (11) while it is at a position at which a photoacoustic image is to be generated as a reference image; and wherein:
the probe position judging means (29) extracts features from the stored reference image, and compares the extracted features of the acoustic wave images.

5. A photoacoustic image generating apparatus (10) as defined in any one of Claims 1 through 4, further comprising:
contact state judging means (31) for judging whether the probe (11) is in contact with the subject; and
light irradiation suppressing means (32) for suppressing output of the light when the contact state judging means (31) judges that the probe (11) is not in contact with the subject.

6. A photoacoustic image generating apparatus (10) as defined in Claim 5, wherein:
the contact state judging means (31) judges whether the probe (11) is in contact with the subject based on reflected acoustic signals detected by the probe (11).

7. A photoacoustic image generating apparatus (10) as defined in any one of Claims 1 through 6, wherein the probe (11) comprises:
a backing material (1);
a plurality of inorganic piezoelectric elements (2) arranged on the surface of the backing material (1);
a first acoustic matching layer (3) provided on the plurality of inorganic piezoelectric elements (2); and
a second acoustic matching layer (4) provided on the first acoustic matching layer (3);
the second acoustic matching layer (4) comprising an upper side organic layer (41) that constitutes a plurality of organic piezoelectric elements (5) and a lower side organic layer (42) that performs acoustic matching with respect to the plurality of inorganic piezoelectric elements (2) with the upper side organic layer (41).

8. A photoacoustic image generating apparatus (10) as defined in any one of Claims 1 through 6, wherein the probe (11) comprises:
a plurality of inorganic piezoelectric elements (2) arranged on a backing material (1); and
a plurality of organic piezoelectric elements (5) provided along with the inorganic piezoelectric elements (2) in a direction parallel to the surface of the backing material (1).

9. A photoacoustic image generating apparatus (10) as defined in either one of Claim 7 and Claim 8, wherein:
the plurality of inorganic piezoelectric elements (2) are employed to transmit acoustic waves; and
the plurality of organic piezoelectric elements (5) are employed to detect reflected acoustic waves.

10. A photoacoustic image generating method, comprising the steps of:
transmitting acoustic waves from a probe (11) toward a subject (step B1);
detecting reflected acoustic signals of the transmitted acoustic waves with the probe (11) (step B2);
comparing the features of reflected acoustic signals detected by the probe (11) against the features of reflected acoustic signals, which are detected in advance by the probe (11) while the probe (11) is at a position at which a photoacoustic image is to be generated, and judging whether the compared features match (step B4);
irradiating light onto the subject (step B6) when it is judged that the compared features match in the comparing step (step B4);
detecting photoacoustic signals generated within the subject due to irradiation of the light with the probe (11) (step B7); and
generating photoacoustic images based on the detected photoacoustic signals (step B8).

11. A photoacoustic image generating method as defined in Claim 10, further comprising the step of:
generating acoustic wave images based on the reflected acoustic signals (step B3); and wherein:
the acoustic wave images are utilized to compare the features of the reflected acoustic signals in the comparing step (step B4).

12. A photoacoustic image generating method as defined in Claim 11, wherein:
features are extracted from the generated acoustic wave images, and the extracted features are compared against the features of the reflected acoustic signals which are obtained in advance in the comparing step (step B4).

13. A photoacoustic image generating method as defined in Claim 12, further comprising the step of:
storing an acoustic wave image generated based on reflected acoustic signals detected by the probe (11) while it is at a position at which a photoacoustic image is to be generated as a reference image; and wherein:
features are extracted from the stored reference image, and the features extracted from the reference image are employed for comparison in the comparing step (B4).

14. A photoacoustic image generating method as defined in any one of Claims 10 through 13, further comprising the steps of:
judging whether the probe (11) is in contact with the subject; and
suppressing output of the light when it is judged that the probe (11) is not in contact with the subject.

15. A photoacoustic image generating method as defined in Claim 14, wherein:
whether the probe (11) is in contact with the subject is judged based on reflected acoustic signals detected by the probe (11).

## Patentansprüche

1. Photoakustische Bilderzeugungsvorrichtung (10), umfassend:
eine Lichtquelleneinheit (13), die auf ein Subjekt aufzustrahlendes Licht ausgibt;
eine Sonde (11), die akustische Wellen auf das Subjekt sendet, innerhalb des Subjekts aufgrund der Bestrahlung mit dem Licht erzeugte photoakustische Signale nachweist und reflektierte akustische Signale der gesendeten akustischen Wellen nachweist;
eine photoakustische Bilderzeugungseinrichtung (26) zum Erzeugen photoakustischer Bilder basierend auf von der Sonde (11) nachgewiesenen photoakustischen Signalen,
**gekennzeichnet durch**
eine Sondenstellungs-Beurteilungseinrichtung (29) zum Vergleichen der Merkmale reflektierter akustischer Signale, die von der Sonde (11) nachgewiesen werden, mit Merkmalen reflektierter akustischer Signale, die von der Sonde (11) vorab nachgewiesen werden, während die Sonde (11) sich in einer Stellung befindet, an der ein photoakustisches Bild zu erzeugen ist, und zum Beurteilen, ob die verglichenen Merkmale übereinstimmen; und
eine Lichtquellensteuereinrichtung (30) zum Anweisen der Lichtquelleneinheit (13), Licht auszugeben, wenn die Sondenstellungs-Beurteilungseinrichtung (29) beurteilt, dass die verglichenen Merkmale übereinstimmen.

2. Photoakustische Bilderzeugungsvorrichtung (10) nach Anspruch 1, weiterhin umfassend:
eine Akustikwellen-Bilderzeugungseinrichtung (26) zum Erzeugen von Akustikwellenbildern basierend auf den reflektierten akustischen Signalen; wobei
die Sondenstellungs-Beurteilungseinrichtung (29) die Akustikwellenbilder dazu verwendet, die Merkmale der reflektierten akustischen Signale zu vergleichen.

3. Photoakustische Bilderzeugungsvorrichtung (10) nach Anspruch 2, bei der
die Sondenstellungs-Beurteilungseinrichtung (29) Merkmale aus den erzeugten Akustikwellenbildern extrahiert und die extrahierten Merkmale mit den Merkmalen derjenigen reflektierten akustischen Signale vergleicht, die vorab erhalten werden.

4. Photoakustische Bilderzeugungsvorrichtung (10) nach Anspruch 3, weiterhin umfassend:
eine Referenzbild-Speichereinrichtung (28) zum Speichern eines Akustikwellenbilds, welches von der Akustikwellenbild-Erzeugungseinrichtung (26) basierend auf von der Sonde (11) nachgewiesenen reflektierten Akustiksignalen erzeugt wird, während die Sonde sich in einer Stellung befindet, in der ein photoakustisches Bild als Referenzbild erzeugt werden soll, und wobei
die Sondenstellungs-Beurteilungseinrichtung (29) Merkmale aus dem gespeicherten Referenzbild extrahiert und die extrahierten Merkmale der Akustikwellenbilder vergleicht.

5. Photoakustische Bilderzeugungsvorrichtung (10) nach einem der Ansprüche 1 bis 4, weiterhin umfassend:
eine Berührzustands-Beurteilungseinrichtung (31) zum Beurteilen, ob sich die Sonde (11) in Berührung mit dem Subjekt befindet; und
eine Lichtbestrahlungs-Unterdrückungseinrichtung (32) zum Unterdrücken der Ausgabe des Lichts, wenn die Berührzustands-Beurteilungseinrichtung (31) beurteilt, dass die Sonde (11) sich nicht in Berührung mit dem Subjekt befindet.

6. Photoakustische Bilderzeugungsvorrichtung (10) nach Anspruch 5, bei der:
die Berührzustands-Beurteilungseinrichtung (31) beurteilt, ob sich die Sonde (11) in Berührung mit dem Subjekt befindet, basierend auf von der Sonde (11) nachgewiesenen reflektierten Akustiksignalen.

7. Photoakustische Bilderzeugungsvorrichtung (10) nach einem der Ansprüche 1 bis 6, bei der die Sonde (11) umfasst:
ein Trägermaterial (1);
mehrere anorganische piezoelektrische Elemente (2), die auf der Oberfläche des Trägermaterials (1) angeordnet sind;
eine erste akustische Anpassschicht (3), die auf den mehreren anorganischen piezoelektrischen Elementen (2) vorgesehen ist; und
eine zweite akustische Anpassschicht (4) auf der ersten akustischen Anpassschicht (3);
wobei die zweite akustische Anpassschicht (4) eine organische Oberseitenschicht (41) aufweist, die mehrere organische piezoelektrische Elemente (5) bildet, und eine organische Unterseitenschicht (42) aufweist, die eine akustische Anpassung bezüglich der mehreren anorganischen piezoelektrischen Elemente (2) mit der organischen Oberseitenschicht (41) bildet.

8. Photoakustische Bilderzeugungsvorrichtung (10) nach einem der Ansprüche 1 bis 6, bei der die Sonde (11) aufweist:
mehrere anorganische piezoelektrische Elemente (2), die auf einem Trägermaterial (1) angeordnet sind; und
mehrere organische piezoelektrische Elemente (5), die zusammen mit den anorganischen piezoelektrischen Elementen (2) in einer Richtung parallel zu der Oberfläche des Trägermaterials (1) vorgesehen sind.

9. Photoakustische Bilderzeugungsvorrichtung (10) nach einem der Ansprüche 7 und 8, bei der:
die mehreren anorganischen piezoelektrischen Elemente (2) dazu dienen, akustische Wellen zu senden; und
die mehreren organischen piezoelektrischen Elemente (5) dazu dienen, reflektierte Akustikwellen nachzuweisen.

10. Photoakustisches Bilderzeugungsverfahren, umfassend folgende Schritte:
Senden von Akustikwellen aus einer Sonde (11) in Richtung eines Subjekts (Schritt B1);
Nachweisen reflektierter Akustiksignale der gesendeten Akustikwellen mit der Sonde (11) (Schritt B2);
Vergleichen der Merkmale der reflektierten Akustiksignale, die von der Sonde (11) nachgewiesen wurden, mit den Merkmalen reflektierter Akustiksignale, die von der Sonde (11) vorab nachgewiesen werden, während die Sonde sich in einer Stellung befindet, in der ein photoakustisches Bild erzeugt werden soll, und Beurteilen, ob die verglichenen Merkmale übereinstimmen (Schritt B4);
Aufstrahlen von Licht auf das Subjekt (Schritt B6), wenn beurteilt wird, dass die verglichenen Merkmale in dem Vergleichsschritt übereinstimmen (Schritt B4);
Nachweisen photoakustischer Signale, die in dem Subjekt erzeugt werden aufgrund des Aufstrahlens des Lichts, mit der Sonde (11) (Schritt (B7); und
Erzeugen photoakustischer Bilder basierend auf den nachgewiesenen photoakustischen Signalen (Schritt B8).

11. Photoakustisches Bilderzeugungsverfahren nach Anspruch 10, weiterhin umfassend den Schritt:
Erzeugen von Akustikwellenbildern basierend auf den reflektierten akustischen Signalen (Schritt B3), und wobei:
die Akustikwellenbilder dazu dienen, die Merkmale der reflektierten akustischen Signale in den Vergleichsschritt zu vergleichen (Schritt B4).

12. Photoakustisches Bilderzeugungsverfahren nach Anspruch 11, bei dem:
Merkmale aus den erzeugten Akustikwellenbildern extrahiert werden und die extrahierten Merkmale mit den Merkmalen der reflektierten Akustiksignale verglichen werden, die zuvor in dem Vergleichsschritt erhalten werden (Schritt B4).

13. Photoakustisches Bilderzeugungsverfahren nach Anspruch 12, weiterhin umfassend den Schritt:
Speichern eines Akustikwellenbilds, welches generiert wird aufgrund von reflektierten Akustiksignalen, die von der Sonde (11) nachgewiesen werden, während sich diese in einer Stellung befindet, in der ein photoakustisches Bild als Referenzbild zu erzeugen ist, und wobei:
Merkmale aus dem gespeicherten Referenzbild extrahiert werden und die aus dem Referenzbild extrahierten Merkmale für den Vergleich in dem Vergleichsschritt (B4) verwendet werden.

14. Photoakustisches Bilderzeugungsverfahren nach einem der Ansprüche 10 bis 13, weiterhin umfassend die Schritte:
Beurteilen, ob die Sonde (11) sich in Berührung mit dem Subjekt befindet; und
Unterdrücken der Ausgabe des Lichts, wenn beurteilt wird, dass die Sonde (11) sich nicht in Berührung mit dem Subjekt befindet.

15. Photoakustisches Bilderzeugungsverfahren nach Anspruch 14, bei dem:
ob die Sonde (11) sich in Berührung mit dem Subjekt befindet, aufgrund von der Sonde (11) nachgewiesener reflektierter Akustiksignale beurteilt wird.

## Revendications

1. Appareil de génération d'images photoacoustiques (10), comprenant :
une unité de source lumineuse (13) qui produit une lumière à irradier sur un sujet ;
une sonde (11) qui transmet des ondes acoustiques vers le sujet, détecte des signaux photoacoustiques générés à l'intérieur du sujet suite à l'irradiation de la lumière, et détecte des signaux acoustiques réfléchis des ondes acoustiques transmises ;
un moyen de génération d'images photoacoustiques (26) destiné à générer des images photoacoustiques sur la base de signaux photoacoustiques détectés par la sonde (11) ;
**caractérisé par**
un moyen de jugement de position de sonde (29) destiné à comparer les particularités de signaux acoustiques réfléchis détectés par la sonde (11) avec les particularités de signaux acoustiques réfléchis, lesquels sont détectés au préalable par la sonde (11) alors que la sonde (11) se trouve en une position où une image photoacoustique doit être générée, et à juger si oui ou non les particularités comparées correspondent, et
un moyen de commande de source lumineuse (30) destiné à ordonner à l'unité de source lumineuse (13) de produire une lumière lorsque le moyen de jugement de position de sonde (29) juge que les particularités comparées correspondent.

2. Appareil de génération d'images photoacoustiques (10) selon la revendication 1, comprenant en outre :
un moyen de génération d'images d'ondes acoustiques (26) destiné à générer des images d'ondes acoustiques sur la base des signaux acoustiques réfléchis, et dans lequel :
le moyen de jugement de position de sonde (29) utilise les images d'ondes acoustiques pour comparer les particularités des signaux acoustiques réfléchis.

3. Appareil de génération d'images photoacoustiques (10) selon la revendication 2, dans lequel
le moyen de jugement de position de sonde (29) extrait des particularités des images d'ondes acoustiques générées, et compare les particularités extraites aux particularités des signaux acoustiques réfléchis qui sont obtenus au préalable.

4. Appareil de génération d'images photoacoustiques (10) selon la revendication 3, comprenant en outre :
un moyen de stockage d'images de référence (28) destiné à stocker une image d'onde acoustique générée par le moyen de génération d'images d'ondes acoustiques (26) sur la base de signaux acoustiques réfléchis détectés par la sonde (11) tandis que celle-ci se trouve en une position où une image photoacoustique doit être générée comme image de référence, et dans lequel :
le moyen de jugement de position de sonde (29) extrait des particularités des images de référence stockées, et compare les particularités extraites des images d'ondes acoustiques.

5. Appareil de génération d'images photoacoustiques (10) selon l'une quelconque des revendications 1 à 4, comprenant en outre :
un moyen de jugement d'état de contact (31) destiné à juger si oui ou non la sonde (11) est en contact avec le sujet, et
un moyen de suppression d'irradiation lumineuse (32) destiné à supprimer la production de la lumière lorsque le moyen de jugement d'état de contact (31) juge que la sonde (11) n'est pas en contact avec le sujet.

6. Appareil de génération d'images photoacoustiques (10) selon la revendication 5, dans lequel
le moyen de jugement d'état de contact (31) juge si oui ou non la sonde (11) est en contact avec le sujet sur la base de signaux acoustiques réfléchis détectés par la sonde (11).

7. Appareil de génération d'images photoacoustiques (10) selon l'une quelconque des revendications 1 à 6, dans lequel la sonde (11) comprend :
une matière absorbante (1) ;
une pluralité d'éléments piézoélectriques inorganiques (2) agencés sur la surface de la matière absorbante (1) ;
une première couche de correspondance acoustique (3) prévue sur la pluralité d'éléments piézoélectriques inorganiques (2), et
une seconde couche de correspondance acoustique (4) prévue sur la première couche de correspondance acoustique (3) ;
la seconde couche de correspondance acoustique (4) comprenant une couche organique latérale supérieure (41) qui constitue une pluralité d'éléments piézoélectriques organiques (5) et une couche organique latérale inférieure (42) qui réalise une correspondance acoustique par rapport à la pluralité d'éléments piézoélectriques inorganiques (2) avec la couche organique latérale supérieure (41).

8. Appareil de génération d'images photoacoustiques (10) selon l'une quelconque des revendications 1 à 6, dans lequel la sonde (11) comprend :
une pluralité d'éléments piézoélectriques inorganiques (2) agencés sur une matière absorbante (1), et
une pluralité d'éléments piézoélectriques organiques (5) prévus le long des éléments piézoélectriques inorganiques (2) dans une direction parallèle à la surface de la matière absorbante (1).

9. Appareil de génération d'images photoacoustiques (10) selon l'une ou l'autre quelconque des revendications 7 et 8, dans lequel :
la pluralité des éléments piézoélectriques inorganiques (2) est utilisée pour transmettre des ondes acoustiques, et
la pluralité des éléments piézoélectriques organiques (5) est utilisée pour détecter des ondes acoustiques réfléchies.

10. Procédé de génération d'images photoacoustiques, comprenant les étapes consistant à :
transmettre des ondes acoustiques d'une sonde (11) vers un sujet (étape B1) ;
détecter des signaux acoustiques réfléchis des ondes acoustiques transmises avec la sonde (11) (étape B2) ;
comparer les particularités de signaux acoustiques réfléchis détectés par la sonde (11) avec les particularités de signaux acoustiques réfléchis, lesquels sont détectés au préalable par la sonde (11) alors que la sonde (11) se trouve en une position où une image photoacoustique doit être générée, et juger si oui ou non les particularités comparées correspondent (étape B4) ;
irradier le sujet avec la lumière (étape B6) lorsqu'il est jugé que les particularités comparées correspondent lors de l'étape de comparaison (étape B4) ;
détecter des signaux photoacoustiques générés à l'intérieur du sujet suite à l'irradiation de la lumière avec la sonde (11) (étape B7), et
générer des images photoacoustiques sur la base des signaux acoustiques détectés (étape B8).

11. Procédé de génération d'images photoacoustiques selon la revendication 10, comprenant en outre l'étape consistant à :
générer des images d'ondes acoustiques sur la base des signaux acoustiques réfléchis (étape B3), et dans lequel :
les images d'ondes acoustiques sont utilisées pour comparer les particularités des signaux acoustiques réfléchis lors de l'étape de comparaison (étape B4).

12. Procédé de génération d'images photoacoustiques selon la revendication 11, dans lequel
les particularités sont extraites des images d'ondes acoustiques générées, et les particularités extraites sont comparées aux particularités des signaux acoustiques réfléchis qui sont obtenus au préalable lors de l'étape de comparaison (étape B4).

13. Appareil de génération d'images photoacoustiques selon la revendication 12, comprenant en outre l'étape consistant à :
stocker une image d'onde acoustique générée sur la base de signaux acoustiques réfléchis détectés par la sonde (11) tandis que celle-ci se trouve en une position où une image photoacoustique doit être générée comme image de référence, et dans lequel :
les particularités sont extraites de l'image de référence stockée, et les particularités extraites de l'image de référence sont utilisées pour une comparaison lors de l'étape de comparaison (B4).

14. Appareil de génération d'images photoacoustiques selon l'une quelconque des revendications 10 à 13, comprenant en outre les étapes consistant à :
juger si oui ou non la sonde (11) est en contact avec le sujet, et à
supprimer la production de la lumière s'il est jugé que la sonde (11) n'est pas en contact avec le sujet.

15. Appareil de génération d'images photoacoustiques selon la revendication 14, dans lequel :
il est jugé si oui ou non la sonde (11) est en contact avec le sujet sur la base de signaux acoustiques réfléchis détectés par la sonde (11).
